# EUROPEAN PATENT APPLICATION

(11) **EP 4 163 292 A1**
(43) Date of publication of application: **12.04.2023**
(21) Application number: 22200274.3
(22) Date of filing: 07.10.2022
(51) Int. Cl.: C07K 14/195

(54) **NOVEL RHODOPSIN MUTANTS**

(30) Priority: 08.10.2021 JP 2021165940
(71) Applicant: Shizuoka Prefectural University Corporation, Shizuoka-shi, Shizuoka 422-8526 (JP)
(72) Inventor: HARA, Kiyotaka, Shizuoka-shi, Shizuoka, 422-8526 (JP); HARA, Yoko, Shizuoka-shi, Shizuoka, 422-8526 (JP); ITO, Sohei, Shizuoka-shi, Shizuoka, 422-8526 (JP); NAKANO, Shogo, Shizuoka-shi, Shizuoka, 422-8526 (JP); SATO, Yoshiki, Shizuoka-shi, Shizuoka, 422-8526 (JP); KAWASAKI, Mayu, Shizuoka-shi, Shizuoka, 422-8526 (JP); MATSUDA, Fumio, Osaka, 5650871 (JP); TOYA, Yoshihiro, Osaka, 5650871 (JP); SEIKE, Taisuke, Osaka, 5650871 (JP); OTSUKA, Kensuke, Osaka, 5650871 (JP)
(74) Representative: Hoffmann Eitle

(57) **Abstract**

The present disclosure provides a mutant of rhodopsin and a protein moiety thereof, nucleic acids encoding them, a cell containing any of them, and a method for culturing the cell.

## Description

### BACKGROUND OF THE INVENTION

### Field of the Invention

The present disclosure relates to a mutant of rhodopsin and a protein moiety thereof, nucleic acids encoding them, a cell containing any of them, and a method for culturing the cell.

Material production using microorganisms, cells, and organisms is a field gaining attention as a production process for chemicals. Patent Literature 1 discloses a yeast expressing delta-rhodopsin (dR) derived from *Haloterrigena turkmenica* in the inner mitochondrial membrane.

Patent Literature 1 demonstrates that the yeast obtained exhibits proton-translocating ability in a light-dependent manner, and the energy production of the yeast is promoted to enhance the production capacity for glutathione, trehalose, and succinic acid. Patent Literature 2 discloses an *Escherichia coli* strain expressing delta-rhodopsin (dR) and that expressing proteorhodopsin (pR) derived from marine picoplankton.

Patent Literature 2 demonstrates that the E. *coli* strains obtained exhibit proton-translocating ability in a light-dependent manner, and the energy production of the *E. coli* strains is promoted to enhance the production capacity for isoprenol. Patent Literature 3 discloses supplying energy to a photoautotroph, such as cyanobacteria, with exogenous opsin introduced therein provides an effect of improving the doubling time, CO₂ fixation rate, and/or formation of carbon-based products. Non Patent Literature 1 demonstrates that expression of opsin in the mitochondria of mammalian cells such as CHO cells allows the cells to be supplied with energy. For example, it is suggested that this effect may prevent cell death due to inhibition of respiratory chain complex I caused by rotenone. Supply of energy to cells is expected to be applied to light-driven biological production.

### Citation List

### Patent Literature

Patent Literature 1: WO 2015/170609A
Patent Literature 2: WO 2020/050113A
Patent Literature 3: WO 2009/062190A

### Non Patent Literature

Non Patent Literature 1: Hara et al., Sci. Rep., 3: 1635, 2013

### SUMMARY OF THE INVENTION

The present invention provides a cell expressing an exogenous opsin and the corresponding rhodopsin. The present invention also provides a method for culturing the cell.

The present inventors obtained various rhodopsins having novel amino acid sequences by modifying a gene encoding rhodopsin. The present invention is based on these.

The present invention provides, for example, the followings.
[1] A rhodopsin or a protein moiety thereof, wherein the rhodopsin:
   (1) has an amino acid sequence set forth in SEQ ID NO: 12, and is capable of exerting proton translocation activity higher than that of a rhodopsin having an amino acid sequence set forth in SEQ ID NO: 9; or
   (2) has an amino acid sequence set forth in SEQ ID NO: 4, and is capable of exerting proton translocation activity higher than that of a rhodopsin having an amino acid sequence set forth in SEQ ID NO: 3; or
   (3) has an amino acid sequence set forth in SEQ ID NO: 8, and is capable of exerting proton translocation activity higher than that of a rhodopsin having an amino acid sequence set forth in SEQ ID NO: 3,
   wherein the protein moiety can be an opsin in a form not binding to all-trans-retinal, and the same applies hereinafter.
[2] The rhodopsin according to [1] or a protein moiety thereof, wherein the rhodopsin has an amino acid sequence set forth in any of SEQ ID NOs: 4, 5 to 7, and 10 to 13.
[3] A nucleic acid, encoding the protein moiety according to [1] or [2].
[4] A cell, comprising the rhodopsin according to [1] or [2] or a protein moiety thereof.
[5] A cell, comprising a nucleic acid encoding the protein moiety according to [1] or [2], wherein preferably the cell optionally further comprises the rhodopsin or protein moiety according to [1] or [2].
[6] A method for culturing the cell according to [4] or [5], the method comprising:
   culturing the cell under culture conditions suitable for culture of the cell.
[7] The method according to [6], wherein the culturing comprises culturing under light irradiation conditions.
[8] A rhodopsin or a protein moiety thereof, wherein the rhodopsin has an amino acid sequence set forth in SEQ ID NO: 4, and is capable of exerting proton translocation activity higher than that of a rhodopsin having an amino acid sequence set forth in SEQ ID NO: 3.
[9] The rhodopsin according to [8] or a protein moiety thereof, wherein
   the amino acid at position 31 of the amino acid sequence set forth in SEQ ID NO: 4 is Q and the amino acid at position 128 of the amino acid sequence set forth in SEQ ID NO: 4 is I;
   the amino acid at position 31 of the amino acid sequence set forth in SEQ ID NO: 4 is A and the amino acid at position 128 of the amino acid sequence set forth in SEQ ID NO: 4 is G; or
   the amino acid at position 31 of the amino acid sequence set forth in SEQ ID NO: 4 is Q and the amino acid at position 128 of the amino acid sequence set forth in SEQ ID NO: 4 is G.
[10] A rhodopsin or a protein moiety thereof, having an amino acid sequence set forth in SEQ ID NO: 5.
[11] A rhodopsin or a protein moiety thereof, having an amino acid sequence set forth in SEQ ID NO: 6.
[12] A rhodopsin or a protein moiety thereof, having an amino acid sequence set forth in SEQ ID NO: 7.
[13] A rhodopsin or a protein moiety thereof, wherein the rhodopsin has an amino acid sequence set forth in SEQ ID NO: 8, and is capable of exerting proton translocation activity higher than that of a rhodopsin having an amino acid sequence set forth in SEQ ID NO: 3.
[14] A rhodopsin or a protein moiety thereof, having an amino acid sequence set forth in SEQ ID NO: 10.
[15] A rhodopsin or a protein moiety thereof, having an amino acid sequence set forth in SEQ ID NO: 11.
[16] A rhodopsin or a protein moiety thereof, wherein the rhodopsin has an amino acid sequence set forth in SEQ ID NO: 12, and is capable of exerting proton translocation activity higher than that of a rhodopsin having an amino acid sequence set forth in SEQ ID NO: 9.
[17] A rhodopsin or a protein moiety thereof, having an amino acid sequence set forth in SEQ ID NO: 13.
[18]
   (A) A rhodopsin or a protein moiety thereof, wherein the rhodopsin or the protein moiety has an amino acid sequence having 90% or higher identity with an amino acid sequence set forth in SEQ ID NO: 10 or 11, wherein the amino acid sequence having 90% or higher identity with the amino acid sequence set forth in SEQ ID NO: 10 or 11 is preferably an amino acid sequence having one to several amino acid mutations, in particular, one or more mutations selected from the group consisting of insertion, deletion, substitution, addition, and removal from the amino acid sequence set forth in SEQ ID NO: 10 or 11, and the rhodopsin is capable of exerting proton translocation activity higher than that of a rhodopsin having an amino acid sequence set forth in SEQ ID NO: 9;
   (B) a rhodopsin or a protein moiety thereof, wherein the rhodopsin or the protein moiety has an amino acid sequence having 90% or higher identity with an amino acid sequence set forth in any of SEQ ID NOs: 5 to 7 and 13, wherein the amino acid sequence having 90% or higher identity with the amino acid sequence set forth in any of SEQ ID NOs: 5 to 7 and 13 is preferably an amino acid sequence having one to several amino acid mutations, in particular, one or more mutations selected from the group consisting of insertion, deletion, substitution, addition, and removal from the amino acid sequence set forth in any of SEQ ID NOs: 5 to 7 and 13, and the rhodopsin is capable of exerting proton translocation activity higher than that of a rhodopsin having an amino acid sequence set forth in SEQ ID NO: 3; or
   (C) a rhodopsin or a protein moiety thereof, wherein the rhodopsin or the protein moiety has an amino acid sequence set forth in SEQ ID NO: 4, wherein the amino acid at position 31 of the amino acid sequence set forth in SEQ ID NO: 4 is Q and the amino acid at position 128 of the amino acid sequence set forth in SEQ ID NO: 4 is G, and the amino acid sequence having 90% or higher identity with the amino acid sequence set forth in SEQ ID NO: 4 is an amino acid sequence having one to several amino acid mutations, in particular, one or more mutations selected from the group consisting of insertion, deletion, substitution, addition, and removal from the amino acid sequence set forth in SEQ ID NO: 4, and the rhodopsin is capable of exerting proton translocation activity higher than that of a rhodopsin having an amino acid sequence set forth in SEQ ID NO: 3.
[19] A rhodopsin or a protein moiety thereof, wherein the rhodopsin or the protein moiety has an amino acid sequence set forth in SEQ ID NO: 8, the amino acid sequence set forth in SEQ ID NO: 8 having one or more mutations selected from the group consisting of (1) to (62) shown later, and is capable of exerting proton translocation activity higher than that of a rhodopsin having an amino acid sequence set forth in SEQ ID NO: 3.
[20] A nucleic acid, encoding the protein moiety according to any one of [8] to [19].
[21] A cell, comprising the rhodopsin according to any one of [8] to [19] or a protein moiety thereof.
[22] A cell, comprising a nucleic acid encoding the protein moiety according to any one of [8] to [19], wherein preferably the cell optionally further comprises the rhodopsin according to any one of [8] to [19] or a protein moiety thereof.
[23] A method for culturing the cell according to [21] or
[22], the method comprising: culturing the cell under culture conditions suitable for culture of the cell.
[24] The method according to [23], wherein the culturing comprises culturing under light irradiation conditions.
[25] A method for identifying or selecting an amino acid sequence or a nucleic acid sequence encoding the amino acid sequence, the method comprising:
   (a1) providing amino acid sequences each having an amino acid sequence having 90% or higher identity with an amino acid sequence set forth in SEQ ID NO: 4, wherein the amino acid at position 31 of the amino acid sequence set forth in SEQ ID NO: 4 is Q and the amino acid at position 128 of the amino acid sequence set forth in SEQ ID NO: 4 is G, and the amino acid sequence having 90% or higher identity with the amino acid sequence set forth in SEQ ID NO: 4 is preferably an amino acid sequence having one to several amino acid mutations, in particular, one or more mutations selected from the group consisting of insertion, deletion, substitution, addition, and removal from said amino acid sequence set forth in SEQ ID NO: 4, and selecting an opsin having proton translocation activity higher than that of an opsin (or rhodopsin) having an amino acid sequence set forth in SEQ ID NO: 3 from opsins (or rhodopsins) having the provided amino acid sequences; or
   (b1) providing amino acid sequences each having an amino acid sequence having 90% or higher identity with an amino acid sequence set forth in any of SEQ ID NOs: 5 to 7 and 13, wherein the amino acid sequence having 90% or higher identity with the amino acid sequence set forth in any of SEQ ID NOs: 5 to 7 and 13 is preferably an amino acid sequence having one to several amino acid mutations, in particular, one or more mutations selected from the group consisting of insertion, deletion, substitution, addition, and removal from the amino acid sequence set forth in any of SEQ ID NOs: 5 to 7 and 13, and selecting an opsin having proton translocation activity higher than that of an opsin (or rhodopsin) having an amino acid sequence set forth in SEQ ID NO: 3 from opsins (or rhodopsins) having the provided amino acid sequences; or
   (c1) providing amino acid sequences each having an amino acid sequence having 90% or higher identity with an amino acid sequence set forth in SEQ ID NO: 10 or 11, wherein the amino acid sequence having 90% or higher identity with the amino acid sequence set forth in SEQ ID NO: 10 or 11 is preferably an amino acid sequence having one to several amino acid mutations, in particular, one or more mutations selected from the group consisting of insertion, deletion, substitution, addition, and removal from the amino acid sequence set forth in SEQ ID NO: 10 or 11, and selecting an opsin having proton translocation activity higher than that of an opsin (or rhodopsin) having an amino acid sequence set forth in SEQ ID NO: 9 from opsins (or rhodopsins) having the provided amino acid sequences.
[26] A method for identifying or selecting an amino acid sequence or a nucleic acid sequence encoding the amino acid sequence, the method comprising:
   (a2) providing an amino acid sequence set forth in SEQ ID NO: 4, and selecting an opsin having proton translocation activity higher than that of an opsin (or rhodopsin) having an amino acid sequence set forth in SEQ ID NO: 3 from opsins (or rhodopsins) each having the provided amino acid sequence; or
   (b2) providing amino acid sequences having one or more mutations selected from the group consisting of (1) to (67) shown later in an amino acid sequence set forth in SEQ ID NO: 8, and selecting an opsin having proton translocation activity higher than that of an opsin (or rhodopsin) having an amino acid sequence set forth in SEQ ID NO: 3 from opsins (or rhodopsins) each having the provided amino acid sequences; or
   (c2) providing an amino acid sequence set forth in SEQ ID NO: 12, and selecting an opsin having proton translocation activity higher than that of an opsin (or rhodopsin) having an amino acid sequence set forth in SEQ ID NO: 9 from opsins (or rhodopsins) each having the provided amino acid sequence.
[27] An opsin obtained by the method according to [25] or [26], or a nucleic acid encoding the opsin.
[28] A cell, comprising the opsin according to [27] or a nucleic acid encoding the opsin.
[29] A method for culturing the cell according to [28], the method comprising:
   culturing the cell under culture conditions suitable for culture of the cell.
[30] The method according to [29], wherein the culturing comprises culturing under light irradiation conditions.
[31] A rhodopsin or a protein moiety thereof, wherein the rhodopsin or the protein moiety has an amino acid sequence set forth in SEQ ID NO: 4 with the amino acid at position 31 being an amino acid other than A.
[32] A rhodopsin or a protein moiety thereof, wherein the rhodopsin or the protein moiety has an amino acid sequence set forth in SEQ ID NO: 4 with the amino acid at position 128 being an amino acid other than I.
[33] A rhodopsin or a protein moiety thereof, wherein the rhodopsin or the protein moiety has an amino acid sequence set forth in SEQ ID NO: 4 with the amino acid at position 31 being Q.
[34] A rhodopsin or a protein moiety thereof, wherein the rhodopsin or the protein moiety has an amino acid sequence set forth in SEQ ID NO: 4 with the amino acid at position 128 being G.
[35] A rhodopsin or a protein moiety thereof, wherein the rhodopsin or the protein moiety has an amino acid sequence set forth in SEQ ID NO: 4 with the amino acid at position 31 being an amino acid other than A and the amino acid at position 128 being an amino acid other than I.
[36] A rhodopsin or a protein moiety thereof, wherein the rhodopsin or the protein moiety has an amino acid sequence set forth in SEQ ID NO: 4 with the amino acid at position 31 being Q and the amino acid at position 128 being G.
[37] A nucleic acid, encoding the protein moiety according to any one of [31] to [36].
[38] A cell, comprising the protein moiety according to any one of [31] to [36].
[39] A cell, comprising a nucleic acid encoding the protein moiety according to any one of [31] to [36], wherein preferably the cell optionally further comprises the rhodopsin or the protein moiety according to any one of [31] to [36].
[40] A method for culturing the cell according to [38] or [39], the method comprising:
   culturing the cell under culture conditions suitable for culture of the cell.
[41] The method according to [40], wherein the culturing comprises culturing under light irradiation conditions. [50] The cell according to [4], [5], [16], [18], [38], or [39], comprising a retinal synthetic system.
[51] A method for culturing the cell according to [50], the method comprising:
   culturing the cell under culture conditions suitable for culture of the cell.
[52] The method according to [51], wherein the culturing comprises culturing under light irradiation conditions.
[70] The cell according to any one of the preceding items, being a prokaryotic cell having the rhodopsin according to any one of the preceding items in the cell membrane.
[71] The cell according to any one of the preceding items, being a eukaryotic cell having the rhodopsin according to any one of the preceding items in the inner mitochondrial membrane.
[72] The rhodopsin or the protein moiety thereof according to any one of the preceding items, wherein the rhodopsin further has a mitochondrial localization signal.
[73] The cell according to any one of the preceding items, being a eukaryotic cell having the rhodopsin according to any one of the preceding items in the inner mitochondrial membrane, wherein the rhodopsin further has a mitochondrial localization signal.
[74] A method for culturing the cell according to any one of [70] to [73], the method comprising:
   culturing the cell under culture conditions suitable for culture of the cell.
[75] The method according to [74], wherein the culturing comprises culturing under light irradiation conditions.
[90] The method according to any one of the preceding items, wherein light irradiation is performed at 2000 µmol photons m⁻²s⁻¹ or less.
[91] The method according to any one of the preceding items, wherein light irradiation is performed at 1000 µmol photons m⁻²s⁻¹ or less.
[92] The method according to any one of the preceding items, wherein light irradiation is performed at 500 µmol photons m⁻²s⁻¹ or less.
[93] The method according to any one of the preceding items, wherein light irradiation is performed at 100 µmol photons m⁻²s⁻¹ or less.

### BRIEF DESCRIPTION OF THE DRAWINGS

FIG. 1 shows the proton translocation activities of MG-E59 mutant and MG-E104 mutant as relative values to the activity of natural delta-rhodopsin;
FIG. 2 shows relative proton translocation activity (left panel) and relative proton translocation activity per cell (right panel) for cells expressing different OP27 mutants to OP27-expressing cells;
FIG. 3 shows relative cell concentration (left panel) and relative proton translocation activity (right panel) for cells expressing different OP27 mutants to OP27-expressing cells;
FIG. 4 shows relative amounts of glutathione produced (left panel) and relative contents of glutathione produced per cell (right panel) for cells expressing different OP27 mutants to OP27-expressing cells;
FIG. 5 shows relative cell concentration (left panel) and relative proton translocation activity (right panel) for cells expressing OP27-A170 mutant to OP27-expressing cells; and
FIG. 6 shows relative amounts of glutathione produced (left panel) and relative contents of glutathione produced per cell (right panel) for cells expressing OP27-A170 mutant to OP27-expressing cells.

### DETAILED DESCRIPTION OF THE PREFERRED EMBODIMENTS

"Rhodopsin" is a complex of the protein opsin and retinal. Classes of rhodopsin include delta-rhodopsin (dR), bacteriorhodopsin (bR) and bR-type rhodopsin (e.g., archaerhodopsin, cruxrhodopsin), sensory rhodopsin I, sensory rhodopsin II, proteorhodopsin (pR) and pR-type rhodopsin, channelrhodopsin I, channelrhodopsin II, halorhodopsin, xanthorhodopsin, sodium-pump-type rhodopsin, and heliorhodopsin. For example, delta-rhodopsin, bacteriorhodopsin, and proteorhodopsin are localized in the cell membranes of prokaryotes, and have a function to pump protons from the inside of a prokaryote to the outside on being placed under light conditions (light irradiation). In prokaryotes, transmembrane proton concentration gradients can be used for ATP production. In eukaryotes, the inner mitochondrial membrane has an oxidative phosphorylation pathway, and forms proton concentration gradients by translocating protons from the intramembrane space to the extramembrane space. Such proton concentration gradients are used for ATP production. Therefore, eukaryotes can be allowed to form proton concentration gradients in a light-dependent manner to promote ATP production by allowing the inner mitochondrial membrane to express opsin.

Herein, "opsin" is a membrane protein of seven-transmembrane structure. Opsin acquires photoreceptivity through binding of retinal, a vitamin A derivative, thereto. Opsins include, for example, opsins listed in Table 1. However, not all of them exert proton-translocating ability in heterologous organisms. Examples of the opsin include, but are not limited to, opsin derived from at least one species selected from the group consisting of *Gloeobacter violaceus, Roseiflexus* sp., *Octadecabacter antarcticus, Photobacterium* sp. SKA34, *Exiguobacterium sibiricum,* the uncultured marine bacterium 66A03, Rhodobacterales bacterium, the uncultured bacterium MedeBAC35C06, the uncultured marine bacterium HF1019P19, and *Psychroflexus torquis.* These opsins have significant proton-translocating ability. Functional variants of opsin capable of forming gradients of concentration of protons in a light-dependent manner are also categorized as opsin.

Herein, a "gene encoding opsin" is a gene encoding opsin operably linked to a regulatory sequence (e.g., a promoter). The regulatory sequence is capable of inducing transcription of opsin in a host (i.e., a cell into which the gene has been introduced). The gene encoding opsin may be codon-optimized for the host. In particular, if heterologous opsin is introduced, codon optimization is preferably performed because of difference in usage of codons across different hosts. The gene encoding opsin can be incorporated in a vector (e.g., a plasmid) with or without an origin of replication suitable for replication in the host. The vector may have an auxotrophic marker and/or drug-selectable marker; if the vector has an auxotrophic marker and/or drug-selectable marker, a host into which a vector has been introduced can be selected with the corresponding nutrient and/or drug. The regulatory sequence may be a constitutive promoter or an inducible promoter. Opsin expressed in a cell and bound to retinal in the intramembrane space is called rhodopsin. The meaning of expressing rhodopsin is having opsin in a form binding to retinal in the intramembrane space. Herein, a "gene encoding rhodopsin" can be used, and the gene is synonymous with a gene encoding opsin.

Herein, organisms are roughly classified into prokaryotes and eukaryotes. Organisms are also roughly classified into unicellular organisms and multicellular organisms. Typically, prokaryotes are unicellular, and there are eukaryotes being unicellular organisms and those being multicellular organisms. Prokaryotes include bacteria, archaeobacteria (archaea), blue-green algae (cyanobacteria), and actinomycetes. Specific examples thereof include coliform bacteria (e.g., *Escherichia coli*), grass bacilli (e.g., *Bacillus subtilis*), lactic acid bacteria (e.g., *Lactobacillus*), acetic acid bacteria (e.g., Acetobacteraceae), coryneform bacteria (*Corynebacterium*), bacteria belonging to the genus *Pseudomonas* (Pseudomonas bacteria), methanogens (e.g., *Methanobacterium*, *Methanosarcina*), the genus *Streptomyces*, the genus *Actinomyces*, and the genus *Agrobacterium.* Eukaryotes include animals, plants, and fungi. Fungi include yeasts, for example, fission yeasts or budding yeasts, such as the genus *Saccharomyces* including *Saccharomyces cerevisiae, Saccharomyces carlsbergensis, Saccharomyces fragilis*, and *Saccharomyces rouxii*, the genus *Shizosaccharomyces* including *Shizosaccharomyces pombe*, the genus *Candida* including *Candida utilis* and *Candida tropicalis*, the genus *Xanthophyllomyces* including *Xanthophyllomyces dendrorhous*, the genus *Pichia*, the genus *Kluyveromyces*, the genus *Yarrowia*, the genus *Hansenula*, the genus *Endomyces*, and the genus *Rhodotorula*. Among them, yeasts belonging to the genus *Saccharomyces*, the genus *Shizosaccharomyces,* the genus *Candida,* the genus *Xanthophyllomyces*, or the genus *Pichia* are preferred, *S. cerevisiae*, which belongs to the genus *Saccharomyces*, *S. pombe*, which belongs to the genus *Shizosaccharomyces,* and *C*. *utilis*, which belongs to the genus *Candida,* are more preferred, and *S. cerevisiae* is the most preferred. Examples include filamentous fungi (e.g., *Aspergillus*, *Trichoderma*, *Humicola*, *Acremonium*, *Fusarium*, *Blakeslea trispora*, and *Penicillium* spp.), insect cells (e.g., cells derived from *Spodoptera frugiperda*, cells derived from *Trichoplusia ni*), *Bombyx mori*, nematodes, plant cells, algae (macroalgae, microalgae), plants (e.g., *Arabidopsis thaliana, Nicotiana tabacum*), and cultured mammalian cells (e.g., Chinese hamster ovary cells (CHO cells), human cells). Cells of an alga (a macroalga, a microalga) may be used. Cells or organisms that perform photosynthesis (phototrophic cells or phototrophic organisms) may be used, and cells or organisms that do not perform photosynthesis (non-phototrophic cells or non-phototrophic organisms) may be used. This is because introduction of opsin promotes formation of proton concentration gradients and thus promotes energy production in any case. Herein, an organism or cell into which a gene encoding opsin is to be introduced is occasionally called a "host".

Herein, the term "exogeneous" means that an object is derived from an organism differing from the host. In typical cases, regulatory sequences can be exogenous. Genes to be expressed can also be exogenous. Accordingly, in a gene operably linked to a regulatory sequence, both of the regulatory sequence and the gene can be exogeneous. The term "endogenous" means that an object is derived from the host itself.

Herein, the "retinal synthetic system" is a multistep pathway to synthesize all-trans-retinal (ATR) from isopentenyl diphosphate (IPP). In the retinal synthetic system, ATR is synthesized from IPP through [1] to [7] in the following.
[1] IPP is converted into dimethylallyl diphosphate (DMAPP) by IPP δ-isomerase.
[2] DMAPP is converted into farnesyl diphosphate (FPP) by FPP synthase (ispA).
[3] FPP is converted into geranylgeranyl pyrophosphate (GGPP) by geranylgeranyl pyrophosphate synthase (crtE).
[4] GGPP is converted into phytoene by phytoene synthase (crtB) .
[5] Phytoene is converted into lycopene by phytoene dehydrogenase (crtI).
[6] Lycopene is converted into β-carotene by lycopene cyclase (crtY).
[7] β-carotene is cleaved by 15,15'-β-carotene dioxygenase (blh) to be converted into two molecules of all-trans-retinal (ATR).

IPP can be produced through the mevalonate pathway or the non-mevalonate pathway.

Herein, "the series of enzymes in the retinal synthetic system" refers to a suit of enzymes in [1] to [7] in the above. Some cells may have an intrinsic enzyme. For example, coliform bacteria and yeasts have IPP δ-isomerase and ispA. Accordingly, if crtE, crtB, crtI, and crtY are added to a coliform bacterium, the coliform bacterium or yeast becomes able to synthesize β-carotene. If blh is further added to the coliform bacterium or yeast, the coliform bacterium or yeast becomes able to synthesize all-trans-retinal. Thus, the meaning of having the series of enzymes in the retinal synthetic system includes having all of the series of enzymes in the retinal synthetic system as a combination of exogenous enzymes and endogenous enzymes. Needless to say, all of the series of enzymes in the retinal synthetic system may be exogenously supplied. Those skilled in the art could appropriately determine which of the enzymes a cell has by analyzing for the presence or absence of each of the corresponding genes or by analyzing gene products. In some embodiments, the host is expressing all of the enzymes selected from the group consisting of IPP δ-isomerase, FPP synthase (ispA), geranylgeranyl pyrophosphate synthase (crtE), phytoene synthase (crtB), phytoene dehydrogenase (crtI), lycopene cyclase (crtY), and 15,15'-β-carotene dioxygenase (blh). In some embodiments, the host has exogenous genes encoding some or all of the enzymes selected from the group consisting of IPP δ-isomerase, FPP synthase (ispA), geranylgeranyl pyrophosphate synthase (crtE), phytoene synthase (crtB), phytoene dehydrogenase (crtI), lycopene cyclase (crtY), and 15,15'-β-carotene dioxygenase (blh) (provided that this is unnecessary for enzymes or genes endogenously possessed by the host).

The present disclosure can provide a rhodopsin having a novel mutation or a protein moiety thereof (i.e., the opsin moiety). The present disclosure can provide a nucleic acid (e.g., DNA or mRNA) encoding a rhodopsin having a novel mutation or a protein moiety thereof (i.e., the opsin moiety). The present disclosure can provide a cell (e.g., a eukaryotic cell or a prokaryotic cell) having the nucleic acid. The present disclosure can provide a method for culturing a cell (e.g., a eukaryotic cell or a prokaryotic cell) having the nucleic acid. The eukaryotic cell can be expressing a rhodopsin having a novel mutation or a protein moiety thereof (i.e., the opsin moiety) on the inner mitochondrial membrane. The prokaryotic cell can be expressing a rhodopsin having a novel mutation or a protein moiety thereof (i.e., the opsin moiety) on the plasma membrane (i.e., the cell membrane).

In some embodiments, the rhodopsin having a novel mutation or a protein moiety thereof (i.e., the opsin moiety) can have an amino acid sequence having 80% or higher sequence identity with an amino acid sequence set forth in SEQ ID NO: 3. The present disclosure can provide a rhodopsin or a protein moiety thereof, the rhodopsin being capable of exerting proton translocation activity higher than that of a rhodopsin having an amino acid sequence set forth in SEQ ID NO: 3. The present disclosure can provide a rhodopsin or a protein moiety thereof, the rhodopsin being capable of imparting cell proliferative capacity higher than that of a rhodopsin having an amino acid sequence set forth in SEQ ID NO: 3 when the rhodopsin or the protein moiety expresses in or on the cell.

The present disclosure can provide a rhodopsin or a protein moiety thereof, the rhodopsin being capable of exerting proton translocation activity higher than that of a rhodopsin having an amino acid sequence set forth in SEQ ID NO: 3. The present disclosure can provide a rhodopsin or a protein moiety thereof, the rhodopsin being capable of imparting cell proliferative capacity higher than that of a rhodopsin having an amino acid sequence set forth in SEQ ID NO: 3 when the rhodopsin or the protein moiety expresses in or on the cell.

According to the present disclosure, the rhodopsin mutant of the present disclosure can have an amino acid sequence set forth in SEQ ID NO: 4. In some embodiments, the amino acid at position 31 of the amino acid sequence set forth in SEQ ID NO: 4 is not A. In some embodiments, the amino acid at position 128 of the amino acid sequence set forth in SEQ ID NO: 4 is not I. In some embodiments, the amino acid at position 31 of the amino acid sequence set forth in SEQ ID NO: 4 is not A, and the amino acid at position 128 is not I. Preferably, the rhodopsin mutant is preferably capable of exerting proton translocation activity higher than that of a rhodopsin having an amino acid sequence set forth in SEQ ID NO: 3. In some embodiments, the amino acid at position 31 of the amino acid sequence set forth in SEQ ID NO: 4 is Q. In some embodiments, the amino acid at position 128 of the amino acid sequence set forth in SEQ ID NO: 4 is G. In some embodiments, the amino acid at position 31 of the amino acid sequence set forth in SEQ ID NO: 4 is Q, and the amino acid at position 128 of the amino acid sequence set forth in SEQ ID NO: 4 is G.

In some embodiments, the rhodopsin mutant of the present disclosure can have an amino acid sequence set forth in SEQ ID NO: 8 (provided that the rhodopsin mutant has a sequence differing from the amino acid sequence set forth in SEQ ID NO: 3).

Description of the sequence possessed by the rhodopsin mutant of the present disclosure in a preferred mode will be provided below. The rhodopsin of the present disclosure in a preferred embodiment has an amino acid sequence set forth in SEQ ID NO: 8, wherein the amino acid sequence set forth in SEQ ID NO: 8 has one or more mutations selected from the group consisting of (1) to (62) in the following (provided that the rhodopsin mutant has a sequence differing from the amino acid sequence set forth in SEQ ID NO: 3):
(1) Xaa at position 1 can be absent or a natural amino acid, and can be preferably absent or M;
(2) the amino acid at position 32 can be A or an amino acid other than A (preferably Q);
(3) the amino acid at position 34 can be N or an amino acid other than N (preferably D);
(4) the amino acid at position 36 can be Y or an amino acid other than Y (preferably F);
(5) the amino acid at position 48 can be L or an amino acid other than L (preferably M);
(6) the amino acid at position 49 can be I or an amino acid other than I (preferably V);
(7) the amino acid at position 51 can be A or an amino acid other than A (preferably S);
(8) the amino acid at position 53 can be A or an amino acid other than A (preferably V);
(9) the amino acid at position 57 can be I or an amino acid other than I (preferably M);
(10) the amino acid at position 59 can be T or an amino acid other than T (preferably A);
(11) the amino acid at position 60 can be T or an amino acid other than T (preferably N or Q);
(12) the amino acid at position 63 can be E or an amino acid other than E (preferably S);
(13) the amino acid at position 64 can be G or an amino acid other than G (preferably A);
(14) the amino acid at position 67 can be K or an amino acid other than K (preferably R);
(15) the amino acid at position 73 can be S or an amino acid other than S (preferably A);
(16) the amino acid at position 79 can be V or an amino acid other than V (preferably I);
(17) the amino acid at position 93 can be S or an amino acid other than S (preferably A);
(18) the amino acid at position 97 can be S or an amino acid other than S (preferably T);
(19) the amino acid at position 108 can be I or an amino acid other than I (preferably L);
(20) the amino acid at position 113 can be L or an amino acid other than L (preferably Q);
(21) the amino acid at position 115 can be V or an amino acid other than V (preferably I);
(22) the amino acid at position 122 can be R or an amino acid other than R (preferably A);
(23) the amino acid at position 124 can be I or an amino acid other than I (preferably V);
(24) the amino acid at position 125 can be T or an amino acid other than T (preferably G);
(25) the amino acid at position 126 can be K or an amino acid other than K (preferably N or A);
(26) the amino acid at position 129 can be I or an amino acid other than I (preferably A);
(27) the amino acid at position 136 can be M or an amino acid other than M (preferably L);
(28) the amino acid at position 140 can be V or an amino acid other than V (preferably L);
(29) the amino acid at position 144 can be V or an amino acid other than V (preferably I);
(30) the amino acid at position 145 can be G or an amino acid other than G (preferably A);
(31) the amino acid at position 148 can be A or an amino acid other than A (preferably L);
(32) the amino acid at position 151 can be I or an amino acid other than I (preferably A);
(33) the amino acid at position 155 can be S or an amino acid other than S (preferably N);
(34) the amino acid at position 156 can be A or an amino acid other than A (preferably V);
(35) the amino acid at position 158 can be L or an amino acid other than L (preferably V);
(36) the amino acid at position 161 can be I or an amino acid other than I (preferably V);
(37) the amino acid at position 165 can be L or an amino acid other than L (preferably A);
(38) the amino acid at position 168 can be A or an amino acid other than A (preferably I or V) or optionally L or an amino acid other than L (preferably A);
(39) the amino acid at position 180 can be S or an amino acid other than S (preferably G);
(40) the amino acid at position 182 can be V or an amino acid other than V (preferably A);
(41) the amino acid at position 183 can be A or an amino acid other than A (preferably S);
(42) the amino acid at position 185 can be D or an amino acid other than D (preferably E);
(43) the amino acid at position 186 can be E or an amino acid other than E (preferably S);
(44) the amino acid at position 188 can be P or an amino acid other than P (preferably S);
(45) the amino acid at position 189 can be A or an amino acid other than A (preferably E);
(46) the amino acid at position 190 can be S or an amino acid other than S (preferably A);
(47) the amino acid at position 192 can be Q or an amino acid other than Q (preferably K);
(48) the amino acid at position 195 can be F or an amino acid other than F (preferably Y);
(49) the amino acid at position 196 can be S or an amino acid other than S (preferably N);
(50) the amino acid at position 197 can be T or an amino acid other than T (preferably A);
(51) the amino acid at position 204 can be I or an amino acid other than I (preferably V);
(52) the amino acid at position 215 can be M or an amino acid other than M (preferably L);
(53) the amino acid at position 219 can be N or an amino acid other than N (preferably A or T);
(54) the amino acid at position 223 can be S or an amino acid other than S (preferably D);
(55) the amino acid at position 225 can be E or an amino acid other than E (preferably A);
(56) the amino acid at position 226 can be A or an amino acid other than A (preferably T);
(57) the amino acid at position 229 can be I or an amino acid other than I (preferably V);
(58) the amino acid at position 233 can be F or an amino acid other than F (preferably L);
(59) the amino acid at position 236 can be V or an amino acid other than V (preferably F);
(60) the amino acid at position 237 can be M or an amino acid other than M (preferably L or V);
(61) the amino acid at position 244 can be V or an amino acid other than V (preferably L); and
(62) the amino acid at position 245 can be I or an amino acid other than I (preferably V).

Description of the sequence possessed by the rhodopsin mutant of the present disclosure in a preferred embodiment will be provided below. The rhodopsin of the present disclosure in a preferred embodiment has an amino acid sequence set forth in SEQ ID NO: 8, wherein the amino acid sequence set forth in SEQ ID NO: 8 has one or more mutations selected from the group consisting of (1A) to (67A) in the following (provided that the rhodopsin mutant has a sequence differing from the amino acid sequence set forth in SEQ ID NO: 3):
(1A) Xaa at position 1 can be absent or a natural amino acid, and can be preferably absent or M;
(2A) the amino acid at position 32 can be A or an amino acid other than A (preferably Q);
(3A) the amino acid at position 34 can be N or an amino acid other than N (preferably D);
(4A) the amino acid at position 36 can be Y or an amino acid other than Y (preferably F);
(5A) the amino acid at position 43 can be L or an amino acid other than L (preferably I);
(6A) the amino acid at position 48 can be L or an amino acid other than L (preferably M);
(7A) the amino acid at position 49 can be I or an amino acid other than I (preferably V);
(8A) the amino acid at position 51 can be A or an amino acid other than A (preferably S);
(9A) the amino acid at position 53 can be A or an amino acid other than A (preferably V);
(10A) the amino acid at position 57 can be I or an amino acid other than I (preferably M);
(11A) the amino acid at position 59 can be T or an amino acid other than T (preferably A);
(12A) the amino acid at position 60 can be T or an amino acid other than T (preferably N or Q or G);
(13A) the amino acid at position 63 can be E or an amino acid other than E (preferably S);
(14A) the amino acid at position 64 can be G or an amino acid other than G (preferably A);
(15A) the amino acid at position 67 can be K or an amino acid other than K (preferably R);
(16A) the amino acid at position 73 can be S or an amino acid other than S (preferably A);
(17A) the amino acid at position 79 can be V or an amino acid other than V (preferably I);
(18A) the amino acid at position 93 can be S or an amino acid other than S (preferably A);
(19A) the amino acid at position 97 can be S or an amino acid other than S (preferably T);
(20A) the amino acid at position 108 can be I or an amino acid other than I (preferably L);
(21A) the amino acid at position 113 can be L or an amino acid other than L (preferably Q);
(22A) the amino acid at position 115 can be V or an amino acid other than V (preferably I);
(23A) the amino acid at position 122 can be R or an amino acid other than R (preferably A);
(24A) the amino acid at position 124 can be I or an amino acid other than I (preferably V);
(25A) the amino acid at position 125 can be T or an amino acid other than T (preferably G);
(26A) the amino acid at position 126 can be K or an amino acid other than K (preferably N or A);
(27A) the amino acid at position 129 can be I or an amino acid other than I (preferably A);
(28A) the amino acid at position 136 can be M or an amino acid other than M (preferably L);
(29A) the amino acid at position 139 can be T or an amino acid other than T (preferably S);
(30A) the amino acid at position 140 can be V or an amino acid other than V (preferably L);
(31A) the amino acid at position 144 can be V or an amino acid other than V (preferably I);
(32A) the amino acid at position 145 can be G or an amino acid other than G (preferably A);
(33A) the amino acid at position 148 can be A or an amino acid other than A (preferably L);
(34A) the amino acid at position 151 can be I or an amino acid other than I (preferably A);
(35A) the amino acid at position 155 can be S or an amino acid other than S (preferably N);
(36A) the amino acid at position 156 can be A or an amino acid other than A (preferably V);
(37A) the amino acid at position 157 can be T or an amino acid other than T (preferably W);
(38A) the amino acid at position 158 can be L or an amino acid other than L (preferably V);
(39A) the amino acid at position 161 can be I or an amino acid other than I (preferably V);
(40A) the amino acid at position 165 can be L or an amino acid other than L (preferably A);
(41A) the amino acid at position 168 can be A or an amino acid other than A (preferably I or V);
(42A) the amino acid at position 180 can be S or an amino acid other than S (preferably G);
(43A) the amino acid at position 182 can be V or an amino acid other than V (preferably A);
(44A) the amino acid at position 183 can be A or an amino acid other than A (preferably S);
(45A) the amino acid at position 185 can be D or an amino acid other than D (preferably E);
(46A) the amino acid at position 186 can be E or an amino acid other than E (preferably S);
(47A) the amino acid at position 188 can be P or an amino acid other than P (preferably S);
(48A) the amino acid at position 189 can be A or an amino acid other than A (preferably E);
(49A) the amino acid at position 190 can be S or an amino acid other than S (preferably A);
(50A) the amino acid at position 192 can be Q or an amino acid other than Q (preferably K);
(51A) the amino acid at position 195 can be F or an amino acid other than F (preferably Y);
(52A) the amino acid at position 196 can be S or an amino acid other than S (preferably N);
(53A) the amino acid at position 197 can be T or an amino acid other than T (preferably A);
(54A) the amino acid at position 200 can be W or an amino acid other than W (preferably L);
(55A) the amino acid at position 204 can be I or an amino acid other than I (preferably V);
(56A) the amino acid at position 215 can be M or an amino acid other than M (preferably L);
(57A) the amino acid at position 219 can be N or an amino acid other than N (preferably A or T or G);
(58A) the amino acid at position 223 can be S or an amino acid other than S (preferably D);
(59A) the amino acid at position 224 can be D or an amino acid other than D (preferably A);
(60A) the amino acid at position 225 can be E or an amino acid other than E (preferably A);
(61A) the amino acid at position 226 can be A or an amino acid other than A (preferably T);
(62A) the amino acid at position 229 can be I or an amino acid other than I (preferably V);
(63A) the amino acid at position 233 can be F or an amino acid other than F (preferably L or V);
(64A) the amino acid at position 236 can be V or an amino acid other than V (preferably F);
(65A) the amino acid at position 237 can be M or an amino acid other than M (preferably L or V);
(66A) the amino acid at position 244 can be V or an amino acid other than V (preferably L); and
(67A) the amino acid at position 245 can be I or an amino acid other than I (preferably V).

The rhodopsin mutant of the present disclosure in a preferred embodiment has an amino acid sequence set forth in SEQ ID NO: 8, wherein the amino acid sequence set forth in SEQ ID NO: 8 can have two or more, three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, 35 or more, 35 or more, 36 or more, 37 or more, 38 or more, 39 or more, 40 or more, 41 or more, 42 or more, 43 or more, 44 or more, 45 or more, 46 or more, 47 or more, 48 or more, 49 or more, 50 or more, 51 or more, 52 or more, 53 or more, 54 or more, 55 or more, 56 or more, 57 or more, 58 or more, 59 or more, or 60 or more features selected from the group consisting of (1) to (62) in the above. The rhodopsin mutant of the present disclosure in a preferred embodiment has an amino acid sequence set forth in SEQ ID NO: 8, wherein the amino acid sequence set forth in SEQ ID NO: 8 can have two or more, three or more, four or more, five or more, six or more, seven or more, eight or more, nine or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, 35 or more, 35 or more, 36 or more, 37 or more, 38 or more, 39 or more, 40 or more, 41 or more, 42 or more, 43 or more, 44 or more, 45 or more, 46 or more, 47 or more, 48 or more, 49 or more, 50 or more, 51 or more, 52 or more, 53 or more, 54 or more, 55 or more, 56 or more, 57 or more, 58 or more, 59 or more, or 60 or more features selected from the group consisting of (1A) to (62A) in the above.

In some embodiments of the present invention, provided is a method comprising selecting an opsin having proton translocation activity higher than that of an opsin having an amino acid sequence set forth in SEQ ID NO: 3 from amino acid sequences set forth in SEQ ID NO: 4.

In some embodiments of the present invention, provided is a method comprising selecting an opsin having proton translocation activity higher than that of an opsin having an amino acid sequence set forth in SEQ ID NO: 3 from opsins each having an amino acid sequence derived from an amino acid sequence set forth in SEQ ID NO: 8 by one or more mutations selected from the group consisting of (1) to (62) in the above. In some embodiments of the present invention, provided is a method comprising selecting an opsin having proton translocation activity higher than that of an opsin having an amino acid sequence set forth in SEQ ID NO: 3 from opsins each having an amino acid sequence derived from an amino acid sequence set forth in SEQ ID NO: 8 by one or more mutations selected from the group consisting of (1A) to (67A) in the above.

In some embodiments of the present invention, provided is a method comprising selecting an opsin having proton translocation activity higher than that of an opsin having an amino acid sequence set forth in SEQ ID NO: 9 from amino acid sequences set forth in SEQ ID NO: 12.

The rhodopsin mutant of the present disclosure in a preferred embodiment has, for example, an amino acid sequence set forth in SEQ ID NO: 12 (provided that the case that the rhodopsin mutant has the amino acid sequence set forth in SEQ ID NO: 9 is excluded). The rhodopsin mutant of the present disclosure in a preferred embodiment has, for example, an amino acid sequence having one or more mutations selected from the group consisting of (1A) to (3A) in the following from an amino acid sequence set forth in SEQ ID NO: 12 (provided that the case that the rhodopsin mutant has the amino acid sequence set forth in SEQ ID NO: 9 is excluded):
(1A) the amino acid at position 125 can be F or an amino acid other than F (preferably L);
(2A) the amino acid at position 142 can be R or an amino acid other than R (preferably H); and
(3A) the amino acid at position 242 can be V or an amino acid other than V (preferably A).

The rhodopsin mutant of the present disclosure in a preferred embodiment can have an amino acid sequence set forth in SEQ ID NO: 5. The rhodopsin mutant of the present disclosure in a preferred embodiment can have an amino acid sequence set forth in SEQ ID NO: 6. The rhodopsin mutant of the present disclosure in a preferred embodiment can have an amino acid sequence set forth in SEQ ID NO: 7.

A rhodopsin and a protein moiety thereof can be provided as the rhodopsin mutant of the present disclosure, wherein the rhodopsin has 90% or higher, 95% or higher, 96% or higher, 97% or higher, 98% or higher, or 99% or higher sequence identity with the above rhodopsin mutant, and is capable of exerting proton translocation activity higher than that of a rhodopsin having an amino acid sequence set forth in SEQ ID NO: 3. A rhodopsin mutant of the present disclosure can have an amino acid sequence having one to several amino acid mutations (preferably one, two, or three amino acid mutations, more preferably three amino acid mutations, even more preferably one amino acid mutation) selected from the group consisting of insertion, deletion, substitution, addition, and removal from the amino acid sequence of the above rhodopsin mutant.

The rhodopsin mutant of the present disclosure can be preferably a fusion protein with a signal sequence.

In a certain preferred embodiment, the rhodopsin mutant of the present disclosure may be linked to a mitochondrial localization signal. Thus, the present disclosure provides an isolated opsin linked to a mitochondrial localization signal, and a gene encoding the opsin. In some embodiments, the rhodopsin mutant of the present disclosure can be a fusion protein containing a mitochondrial localization signal and an opsin, wherein the two are directly or indirectly linked together via a peptide bond. Once being expressed in a eukaryotic cell or eukaryote, the opsin linked to a mitochondrial localization signal migrates into mitochondria. Thus, a eukaryotic cell or eukaryote having a rhodopsin expressed in mitochondria can be obtained. The rhodopsin expressed in mitochondria can promote ATP synthesis by forming proton concentration gradients between the inside and outside of the inner mitochondrial membrane. Hence, the opsin linked to a mitochondrial localization signal is suitable for expressing in eukaryotic cells or eukaryotes. Any mitochondrial localization signal well known to those skilled in the art can be used, such as the ALA synthase Hem1, S2 peptide, or oligoarginine (e.g., octaarginine), peroxiredoxin PRX1, pyruvate dehydrogenase β subunit, the heat-shock protein HSP60, γ subunit of ATP synthase, α subunit of ATP synthase, subunit 2, subunit 4, and subunit 6 of cytochrome c oxidase, succinate dehydrogenase, cytochrome b small subunit, citrate synthase 1 (CIT1), acetolactate synthase catalytic subunit (ILV2), ketol-acid reductoisomerase, mitofilin, and cytochrome-c-degrading enzyme (Sue). The opsin can be localized in mitochondria also by allowing the opsin to be translated in mitochondria, even without a mitochondrial localization signal. If a codon for tryptophan is changed to TAA, for example, such an opsin can be translated exclusively in mitochondria. TGA is a stop codon in translation in the cytoplasm, and codes for tryptophan in translation in mitochondria. TAA can be used as a stop codon in mitochondria. Accordingly, in some embodiments, the gene encoding opsin can contain TGA as a codon coding for tryptophan. The above rhodopsin mutant can be expressed in prokaryotic cells in such a manner that the rhodopsin mutant is expressed in the plasma membrane.

The present disclosure provides a nucleic acid encoding the above rhodopsin mutant. The nucleic acid can be, for example, DNA or mRNA.

Preferably, the DNA encoding the above rhodopsin mutant is operably linked to a regulatory sequence (e.g., a promoter). The regulatory sequence is capable of inducing transcription of opsin in a host (a cell into which the gene has been introduced). The gene encoding opsin may be codon-optimized for the host. In particular, if heterologous opsin is introduced, codon optimization is preferably performed because of difference in usage of codons across different hosts. The gene encoding opsin can be incorporated in a vector (e.g., a plasmid) with or without an origin of replication suitable for replication in the host. The vector may have an auxotrophic marker and/or drug-selectable marker; if the vector has an auxotrophic marker and/or drug-selectable marker, a host into which a vector has been introduced can be selected with the corresponding nutrient and/or drug. The regulatory sequence may be a constitutive promoter or an inducible promoter. Opsin expressed in a cell and bound to retinal in the membrane is called rhodopsin. The meaning of expressing rhodopsin is having opsin in a form binding to retinal in the membrane.

The regulatory sequence is, for example, a promoter. Promoters include class I promoters (applicable to transcription of pre-rRNA), class II promoters (including a core promoter and upstream promoter elements, and being applicable to transcription of mRNA), and class III promoters (further classified into types I, II, and III). The regulatory sequence, also called a control sequence, may be any promoter capable of transcribing mRNA in cells such as animal cells and plant cells. For example, various pol II promoters can be used as a first regulatory sequence. The pol II promoters include, but are not limited to, the CMV promoter, EF1 promoter (EF1α promoter), SV40 promoter, MSCV promoter, hTERT promoter, β-actin promoter, CAG promoter, and CBh promoter. In addition, promoters that drive bacteriophage-derived RNA polymerase, such as the T7 promoter, T3 promoter, and SP6 promoter, and pol III promoters such as the U6 promoter can be included as the promoter. The T7 promoter is preferably used for transcription from circular DNA, and the SP6 promoter is preferably used for transcription from linear DNA. The promoter may be an inducible promoter. An inducible promoter is a promoter being capable of inducing expression of a polynucleotide operably linked to the promoter only in the presence of an inducer that drives the promoter. Some inducible promoters are those being capable of inducing expression of a polynucleotide operably linked to the promoter only in the absence of an inhibitor that suppresses the activity of the promoter. Inducible promoters include promoters that induce gene expression through heating such as heat-shock promoters, but are not limited thereto. Promoters that are inducible include promoters that are inducible with a drug. Such promoters that are drug-inducible include the cumate operator sequence, lambda operator sequences (e.g., 12 × lambda Op), and promoters that are tetracycline-inducible. Tetracycline-inducible promoters include promoters that drive gene expression in the presence of tetracycline or a derivative thereof (e.g., doxycycline) or reverse tetracycline-controlled transactivator (rtTA). An example of tetracycline-inducible promoters is the TRE3G promoter.

The mRNA encoding the above rhodopsin mutant can have a 5' untranslated region (UTR) and a 3' UTR. The mRNA can have Cap structure at the 5' end (Furuichi Y & Miura K. Nature. 1975; 253 (5490): 374-5). Cap structure is capable of adding CapO structure to mRNA with an Anti-Reverse Cap Analogues (ARCA) method using a Cap analog (Stepinski J et al. RNA. 2001 Oct; 7 (10): 1486-95). Additional 2'-O-methyltransferase treatment allows the CapO structure of mRNA to convert into Cap1 structure. Those operations can be performed by using a conventional method, for example, a commercially available kit such as a ScriptCap m7G Capping System and a ScriptCap 2'-O-Methyltransferase Kit, or by using a T7 mScript Standard mRNA Production System (AR Brown CO., LTD), as well. The mRNA can have a poly A chain. Addition of a poly A chain can be performed by using a conventional method, for example, an A-Plus Poly (A) Polymerase Tailing Kit (AR Brown CO., LTD). Accordingly, in some embodiments, the mRNA can be an mRNA having Cap structure at the 5' end and poly A at the 3' end in which at least some or all of the uridine residues are pseudouridine (preferably, 1-methylpseudouridine) residues. The mRNA can be an isolated mRNA or a synthesized mRNA.

The present disclosure provides a cell (e.g., a eukaryotic cell or a prokaryotic cell) comprising a nucleic acid encoding the above rhodopsin mutant. The cell can be any of the aforementioned hosts. The eukaryotic cell can be expressing a rhodopsin having a novel mutation or a protein moiety thereof (i.e., the opsin moiety) on the inner mitochondrial membrane. The prokaryotic cell can be expressing a rhodopsin having a novel mutation or a protein moiety thereof (i.e., the opsin moiety) on the plasma membrane (i.e., the cell membrane).

According to the present disclosure, the cell can be cultured under conditions suitable for culture of the cell. According to the present disclosure, the cell can be proliferated by culturing under conditions suitable for culture of the cell. The conditions suitable for culture of the cell are composed of various parameters suitable for culture of the cell, such as medium, temperature (e.g., from room temperature to 37°C), carbon dioxide concentration (e.g., approximately 5%), oxygen concentration (e.g., approximately 10% to 25%, such as approximately 20%), and time, and those skilled in the art could appropriately determine the parameters. According to the present disclosure, the cell can be cultured under conditions suitable for culture of the cell and under light irradiation conditions (see WO 2015/170609A, WO 2020/050113A, WO 2009/062190A, and Hara et al., Sci. Rep., 3: 1635, 2013; the entire of each is incorporated herein by reference).

The cell (i.e., the host) can be a prokaryote or a eukaryote. The host can be a unicellular organism or a multicellular organism. The host can be a microorganism. The host can be a phototrophic cell or a phototrophic organism. The host can be a non-phototrophic cell or a non-phototrophic organism. The host can be an organism selected from the group consisting of bacteria, archaeobacteria (archaea), blue-green algae (cyanobacteria), and actinomycetes. In some embodiments, the host can be an organism selected from the group consisting of coliform bacteria (e.g., *Escherichia coli*), grass bacilli (e.g., *Bacillus subtilis*), lactic acid bacteria (e.g., *Lactobacillus*), acetic acid bacteria (e.g., Acetobacteraceae), coryneform bacteria (*Corynebacterium*), bacteria belonging to the genus *Pseudomonas* (Pseudomonas bacteria), methanogens (e.g., *Methanobacterium*, *Methanosarcina*), the genus *Streptomyces*, the genus *Actinomyces*, and the genus *Agrobacterium.* In some embodiments, the host can be an organism selected from the group consisting of animals, plants, and fungi. In some embodiments, the host can be a yeast. In some embodiments, the host can be a fission yeast or a budding yeast. In some embodiments, the host can be a yeast selected from the group consisting of the genus *Saccharomyces* such as *Saccharomyces cerevisiae*, *Saccharomyces carlsbergensis*, *Saccharomyces fragilis*, and *Saccharomyces rouxii*, the genus *Shizosaccharomyces* such as *Shizosaccharomyces pombe*, the genus *Candida* such as *Candida utilis* and *Candida tropicalis*, the genus *Xanthophyllomyces* such as *Xanthophyllomyces dendrorhous*, the genus *Pichia*, the genus *Kluyveromyces*, the genus *Yarrowia*, the genus *Hansenula*, the genus *Rhodotorula*, and the genus *Endomyces.* In some embodiments, the host can be selected from the group consisting of filamentous fungi (e.g., *Aspergillus*, *Trichoderma*, *Humicola*, *Acremonium*, *Fusarium*, *Blakeslea trispora,* and *Penicillium* spp.), insect cells (e.g., cells derived from *Spodoptera frugiperda*, cells derived from *Trichoplusia ni*), nematodes, *Bombyx mori,* plant cells, algae (macroalgae, microalgae), plants (*Arabidopsis thaliana*, *Nicotiana tabacum*), and cultured mammalian cells (e.g., Chinese hamster ovary cells (CHO cells), human cells). The host may be an alga (a macroalga, a microalga). The opsin to be introduced into the host can be exogenous. The host and the opsin can be heterologous to each other. The animal can be non-human. If the host is a prokaryote, the opsin can be expressed on the cell membrane. If the host is a eukaryote, the opsin can be expressed on the inner mitochondrial membrane. Techniques well known to those skilled in the art for expression can be used as a method therefor.

The host can have all of the series of enzymes in the retinal synthetic system in addition to a gene encoding exogenous opsin. This allows reduction of the amount of retinal to be added to the medium if the host is capable of self-synthesizing retinal. If the amount of retinal synthesized by the host is enough, it is not needed to add retinal to the medium. If the host lacks some of the series of enzymes in the retinal synthetic system, exogenous genes encoding the enzymes not possessed by the host can be introduced into the host. The exogenous genes encoding the enzymes not possessed by the host can be each a gene operably linked to a regulatory sequence, and introduced into an expression cassette in a vector. Those skilled in the art could appropriately determine whether the host has an enzyme through biochemical analysis (e.g., Western blotting) or genetic analysis (e.g., sequencing or PCR).

The host with expression of rhodopsin can be cultured under culture conditions suitable for the host. Those skilled in the art could appropriately determine the culture conditions. The host with expression of rhodopsin can be cultured under light irradiation conditions. Culturing under light irradiation promotes ATP synthesis in the host with expression of rhodopsin, and energy is to be supplied by the light irradiation. The light irradiation may be any irradiation to a degree that allows rhodopsin to receive light, and the intensity can be, for example, but not limited to, 0.01 µmol photons m⁻²s⁻¹ or more, 0.02 µmol photons m⁻²s⁻¹ or more, 0.03 µmol photons m⁻²s⁻¹ or more, 0.04 µmol photons m⁻²s⁻¹ or more, or 0.05 µmol photons m⁻²s⁻¹ or more. The light irradiation can be in an intensity of 0.1 µmol photons m⁻²s⁻¹ or less, 0.2 µmol photons m⁻²s⁻¹ or less, 0.3 µmol photons m⁻²s⁻¹ or less, 0.4 µmol photons m⁻²s⁻¹ or less, 0.5 µmol photons m⁻²s⁻¹ or less, 0.6 µmol photons m⁻²s⁻¹ or less, 0.7 µmol photons m⁻²s⁻¹ or less, 0.8 µmol photons m⁻²s⁻¹ or less, 0.9 µmol photons m⁻²s⁻¹ or less, 1.0 µmol photons m⁻²s⁻¹ or less, 1.1 µmol photons m⁻²s⁻¹ or less, 1.2 µmol photons m⁻²s⁻¹ or less, 1.3 µmol photons m⁻²s⁻¹ or less, 1.4 µmol photons m⁻²s⁻¹ or less, 1.5 µmol photons m⁻²s⁻¹ or less, 1.6 µmol photons m⁻²s⁻¹ or less, 1.7 µmol photons m⁻²s⁻¹ or less, 1.8 µmol photons m⁻²s⁻¹ or less, 1.9 µmol photons m⁻²s⁻¹ or less, 2 µmol photons m⁻²s⁻¹ or less, 3 µmol photons m⁻²s⁻¹ or less, 4 µmol photons m⁻²s⁻¹ or less, 5 µmol photons m⁻²s⁻¹ or less, 6 µmol photons m⁻²s⁻¹ or less, 7 µmol photons m⁻²s⁻¹ or less, 8 µmol photons m⁻²s⁻¹ or less, 9 µmol photons m⁻²s⁻¹ or less, 10 µmol photons m⁻²s⁻¹ or less, 15 µmol photons m⁻²s⁻¹ or less, 20 µmol photons m⁻²s⁻¹ or less, 20 µmol photons m⁻²s⁻¹ or less, 30 µmol photons m⁻²s⁻¹ or less, 40 µmol photons m⁻²s⁻¹ or less, 50 µmol photons m⁻²s⁻¹ or less, 60 µmol photons m⁻²s⁻¹ or less, 70 µmol photons m⁻²s⁻¹ or less, 80 µmol photons m⁻²s⁻¹ or less, 90 µmol photons m⁻²s⁻¹ or less, 100 µmol photons m⁻²s⁻¹ or less, 110 µmol photons m⁻²s⁻¹ or less, 120 µmol photons m⁻²s⁻¹ or less, 130 µmol photons m⁻²s⁻¹ or less, 140 µmol photons m⁻²s⁻¹ or less, 150 µmol photons m⁻²s⁻¹ or less, 160 µmol photons m⁻²s⁻¹ or less, 170 µmol photons m⁻²s⁻¹ or less, 180 µmol photons m⁻²s⁻¹ or less, 190 µmol photons m⁻²s⁻¹ or less, 200 µmol photons m⁻²s⁻¹ or less, 300 µmol photons m⁻²s⁻¹ or less, 400 µmol photons m⁻²s⁻¹ or less, 500 µmol photons m⁻²s⁻¹ or less, 600 µmol photons m⁻²s⁻¹ or less, 700 µmol photons m⁻²s⁻¹ or less, 800 µmol photons m⁻²s⁻¹ or less, 900 µmol photons m⁻²s⁻¹ or less, 1000 µmol photons m⁻²s⁻¹ or less, 1500 µmol photons m⁻²s⁻¹ or less, or 2000 µmol photons m⁻²s⁻¹ or less. The light for irradiation may be from any of a light bulb (e.g., a filament lamp, a halogen lamp), a fluorescent lamp, a high-pressure discharge lamp, a low-pressure discharge lamp, an LED, EL, chemiluminescence, bioluminescence, and sunlight, and light with a wavelength of 300 to 800 nm or 450 to 650 nm, such as 550 nm, can be used. The light irradiation may be performed continuously or intermittently.

Providing the host with energy by light irradiation allows the host to reduce the consumption of carbon sources for energy production. Providing the host with energy by light irradiation allows the host to use larger amounts of raw materials such as carbon sources in pathways involved in material production (e.g., the pentose phosphate pathway or the like). Therefore, light irradiation makes the condition of the host suitable for material production. Thus, material production can be performed in a preferable manner by supplying raw materials such as carbon sources to the host.

A microorganism with expression of rhodopsin can exhibit low-pH resistance and/or high-temperature resistance under light irradiation conditions.

The term low-pH resistance means that, in an environment at low pH (e.g., lower than pH 5.0) that lowers the abilities of a host (one or more abilities selected from the group consisting of proliferative capacity, ATP synthetic capacity, and material production capacity), one or more abilities selected from the group consisting of proliferative capacity, ATP synthetic capacity, and material production capacity become higher under light irradiation conditions than those without the irradiation, and/or become higher under conditions with expression of rhodopsin than those without the expression. For yeasts, for example, the term low-pH resistance means that, under a pH condition of lower than pH 5.0, pH 4.5 or lower, or pH 4.0 or lower, such as pH 3.2 to 3.7 (e.g., pH 3.5), one or more abilities selected from the group consisting of proliferative capacity, ATP synthetic capacity, and material production capacity become higher than the corresponding abilities under conditions without irradiation and/or under conditions without expression of rhodopsin. The host having low-pH resistance through expression of rhodopsin can be cultured in a preferable manner even under low-pH conditions. One or more abilities selected from the group consisting of proliferation, ATP synthetic capacity, and material production capacity are suppressed in other contaminating microorganisms under low-pH conditions, which can allow the host to be selectively cultured.

The term high-temperature resistance means that, in a high-temperature environment that lowers the abilities of a host (one or more abilities selected from the group consisting of proliferative capacity, ATP synthetic capacity, and material production capacity), one or more abilities selected from the group consisting of proliferative capacity, ATP synthetic capacity, and material production capacity become higher under light irradiation conditions than those without the irradiation, and/or become higher under conditions with expression of rhodopsin than those without the expression. For yeasts, for example, the term high-temperature resistance means that, under a condition of 32°C or higher, 33°C or higher, or 34°C or higher, such as 34°C to 36°C (e.g., 35°C) or higher, one or more abilities selected from the group consisting of proliferative capacity, ATP synthetic capacity, and material production capacity become higher than the corresponding abilities under conditions without irradiation and/or under conditions without expression of rhodopsin. The proliferation of other contaminating microorganisms is suppressed under high-temperature conditions, which can allow the host to be selectively cultured.

A microorganism with expression of rhodopsin can have low-pH resistance and high-temperature resistance under light irradiation conditions. In this case, the host having low-pH resistance and high-temperature resistance through expression of rhodopsin can be cultured in a preferable manner under low-pH and high-temperature conditions. One or more abilities selected from the group consisting of proliferation, ATP synthetic capacity, and material production capacity are suppressed in other contaminating microorganisms under low-pH and high-temperature conditions, which can allow the host to be selectively cultured.

Material production can be performed in a preferable manner by culturing a cell or organism expressing rhodopsin under the aforementioned conditions. To increase the amount of a specific material to be produced, the cell or organism expressing rhodopsin can be cultured under conditions suitable for production of the material. The culture conditions can satisfy either one or both of low-pH conditions and high-temperature conditions. To increase the amount of a specific material to be produced, additional modification for the cell or organism with expression of rhodopsin is permitted. Examples of such modification include: modification to mutate and/or introduce an enzyme involved in a production pathway for the material of interest; destruction of an enzyme involved in the decomposition of the material of interest, lowering of the expression level of the enzyme, and deletion or lowering of the activity of the enzyme; and/or destruction of an enzyme involved in a competing pathway, lowering of the expression level of the enzyme, and/or deletion or lowering of the activity of the enzyme.

Materials to be produced through material production can include organic acids, peptides, amino acids, proteins, nucleosides, vitamins, saccharides, sugar alcohols, alcohols, isoprenoids, and lipids. Specific examples are as follows. Examples of the organic acids can include acetic acid, lactic acid, succinic acid, and α-ketoacid (2-oxoacid). Examples of the peptides can include glutathione, alanyl-glutamine, and γ-glutamyl-valyl-glycine, and examples of the polypeptides can include polylysine and polyglutamic acid. Examples of the amino acids can include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophan, L-phenylalanine, L-serine, L-cysteine, L-3-hydroxyproline, L-4-hydroxyproline, and 5-aminolevulinic acid. Examples of the proteins can include luciferase, inosine kinase, glutamate 5-kinase (EC 2.7.2.11), glutamate-5-semialdehyde dehydrogenase (EC 1.2.1.41), pyrroline-5-carboxylate reductase (EC 1.5.1.2), γ-glutamylcysteine synthetase (EC 6.3.2.2), glutathione synthetase (EC 6.3.2.3), human granulocyte colony-stimulating factor, xylose reductase, and P450. Examples of the nucleosides can include inosine, guanosine, inosinate, guanylate, and adenylate. Examples of the vitamins can include riboflavin, thiamine, and ascorbic acid. Examples of the saccharides can include xylose and mannose, examples of the sugar alcohols can include xylitol and mannitol, and examples of the alcohols can include ethanol. Examples of the isoprenoids can include mevalonate (MVA), isoprenol, astaxanthin, isoprene, isopentenol, limonene, pinene, farnesene, and bisabolene. Examples of the lipids can include propionic acid, hydroxypropionic acid, EPA (eicosapentaenoic acid), and DHA (docosahexaenoic acid). Particularly preferred as useful materials in the present invention are acetic acid as organic acid, glutathione as peptide, mevalonate and isoprenol as isoprenoid, and hydroxypropionic acid as lipid. Examples of the materials to be produced include ATP, glutathione, and isoprenoid.

### Examples

### Example 1: Random Mutation of dR

For a delta-rhodopsin (dR) gene (753 bp; SEQ ID NO: 9), conditions for error-prone PCR to introduce about two mutations per 1 kb of DNA in the dR gene part of pCDF-dR were set in accordance with a method by Leung DW et al. (Leung DW et al., (1989) Biotechniques, 1 (1): 11-15). Inverse PCR for a vector was performed under recommended conditions written in a manual attached to DNA polymerase (KOD plus neo) used. Amplified DNA fragments were purified with a PureLink^{™} PCR Purification Kit (Invitrogen), and ligation was then performed by using Gibson Assembly Master Mix (NEW ENGLAND BioLabs). With use of the resulting library of mutated plasmids of pCDFDuet-dR with random mutations, MG1655 strain was transformed through the heat-shock method. The MG1655 strain was applied onto LB-streptomycin plate medium and cultured at 37°C overnight to afford approximately 7000 strains of transformants. Appropriate amounts of milliQ sterile water was added to the surface of the plate, colonies were all mixed together to prepare bacterial cell suspensions containing the transformants, and the bacterial cell suspensions were each mixed with the same volume of 50% glycerol to prepare glycerol stocks.

Each of the resulting glycerol stocks of the library of randomly mutated strains of dR was applied onto LB-streptomycin plate medium and cultured at 37°C overnight. Single colonies formed were each inoculated into 5 mL of LB-streptomycin liquid medium, and cultured at 150 rpm overnight. The resulting culture solution was inoculated into 50 mL of M9-streptomycin selective medium in a 200-mL flask to reach OD₆₀₀ = 0.05, and cultured at 37°C and 150 rpm overnight. The resulting culture solution was inoculated into 50 mL of fresh M9-streptomycin selective medium to reach OD₆₀₀ = 0.05 and cultured at 37°C and 150 rpm for 4 hours, 10 µM all-trans-retinal and 25 µM isopropyl-β-D-thiogalactopyranoside (IPTG) were then added, and the resultant was cultured at 37°C and 150 rpm in a dark place for 24 hours.

Subsequently, screening for salt tolerance was performed under light irradiation conditions at 50 µE. Specifically, 30 mL of each culture solution was centrifuged at 6000 rpm for 2 minutes, and the supernatant was discarded. With addition of 2 mL of unbuffered solution for measurement of proton pump activity (10 mM NaCl, 10 mM MgSO₄, 0.1 mM CaCl₂), each residue was gently suspended. Each of the resulting bacterial cell suspensions was centrifuged again at 6000 rpm for 2 minutes, and the supernatant was discarded. The bacterial cell pellets were suspended in unbuffered solution for measurement of proton pump activity to reach OD₆₀₀ = 0.5. The resulting bacterial cell suspensions were each mixed with the same volume of 50% glycerol to prepare glycerol stocks. For further concentrating strains with high proton translocation activity, the second round of screening for salt tolerance was performed for the glycerol stocks with the same method. Similarly, bacterial cell suspensions after completion of the second round of screening for salt tolerance were each mixed with the same volume of 50% glycerol to prepare glycerol stocks.

Each of the bacterial cell suspensions after completion of screening for salt tolerance was applied onto LB-streptomycin selective plate medium and cultured at 37°C overnight, and 110 strains were randomly selected from the resulting single colonies, and each inoculated into 2 mL of LB-streptomycin selective medium and cultured at 150 rpm overnight. Subsequently, the resultants were each subcultured in 5 mL of M9-streptomycin selective medium to reach OD₆₀₀ = 0.05, and cultured at 150 rpm overnight. Then, the resultants were each subcultured in 5 mL of fresh M9-streptomycin selective medium to reach OD₆₀₀ = 0.05, and cultured at 150 rpm for 4 hours. Thereafter, with addition of all-trans-retinal and IPTG to reach final concentrations of 10 µM and 25 µM, respectively, culture was performed at 150 rpm in a dark place overnight. The culture solutions were observed for color, and 21 bacterial strains with particularly intense pink color, which is characteristic to dR, were selected.

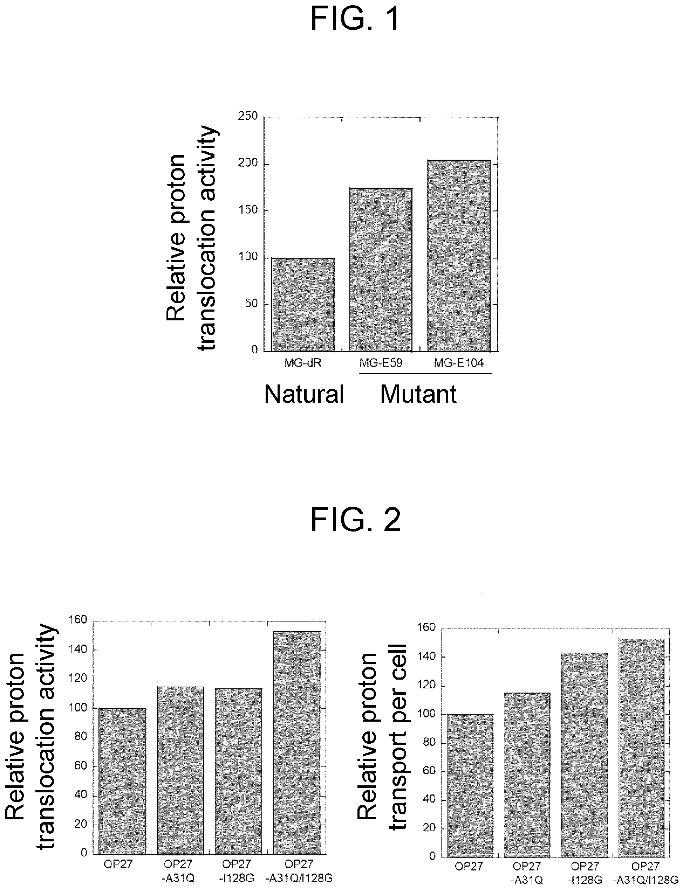

The 21 strains selected were cultured, and subjected to measurement of proton pump activity. The results are represented as ratios of the proton translocation activity of a mutant to that of the natural form, which is assumed as 100 (relative proton translocation activity). As shown in FIG. 1, the results confirmed higher proton translocation activity for the MG-E59-expressing strain and the MG-E104-expressing strain than the natural MG-dR-expressing strain (FIG. 1).

These bacterial cells were each inoculated into 5 mL of LB-streptomycin selective medium, and the resulting bacterial cells were subjected to plasmid extraction using a Labo Pass^{™} Mini (COSMO Genetech), and sequence analysis was performed for the sequence of the dR gene part. Table 1 shows the sequences of primers used for the sequence analysis.

### [Table 1]

**Table 1: Sequences of primers**

| Primer name | Sequence (from 5'to 3') | SEQ ID NO |
|---|---|---|
| dR_colony_F3 forward | CGTATTGTACACGGCCGCATAATC | 1 |
| dR_colony_R3 reverse | TATGCTAGTTATTGCTCAGCGGTGG | 2 |

MG-E59 strain and MG-E104 strain were both confirmed to have undergone amino acid mutation. Table 2 shows mutations identified in dR of MG-E104 and MG-E59 strains (SEQ ID NOs: 10 and 11, respectively), which exhibited high proton translocation rates.

### [Table 2]

**Table 2: Table 2: Mutations in DNA sequences (amino acid sequences) of different mutated clone strains**

| MG-E104 | | MG-E59 | |
|---|---|---|---|
| 2 | | 3 | |
| T373C | (F125L) | T663C | (D221D) |
| G425A | (R142H) | A672G | (A224A) |
| | | T725C | (V242A) |

### Example 2: Site-Specific Mutation of OP27

OP27-A31Q, OP27-I128G, and OP27-A31Q/I128G (having both A31Q and I128G) (SEQ ID NO: 4, wherein X₁ is Q, and/or X₂ is G), which were site-specific mutants of the rhodopsin OP27, were each introduced into *Escherichia coli.* Specifically, nucleic acids (DNAs) encoding OP27 and OP27-A31Q, OP27-I128G, and OP27-A31Q/I128G were each introduced into a pCDF-Duet1 vector to prepare pCDF-OP27 and pCDF-OP27-A31Q, pCDF-OP27-I128G, and pCDF-OP27-A31Q/I128G. An *E. coli* MG1655(DE3) strain was transformed through the heat-shock method using pCDF-OP27, pCDF-OP27-A31Q, pCDF-OP27-I128G, or pCDF-OP27-A31Q/I128G. The *E. coli* strain obtained by introducing pCDF-OP27 into the *E. coli* MG1655(DE3) strain is called "OP27-expressing *E. coli* strain", and was used as a control strain. The *E*. *coli* MG1655(DE3) transformants into which pCDF-OP27-A31Q, pCDF-OP27-I128G, or pCDF-OP27-A31Q/I128G was introduced are called "pCDF-OP27-A31Q-expressing *E. coli* strain", "pCDF-OP27-I128G-expressing *E. coli* strain", and "pCDF-OP27-A31Q/I128G-expressing *E. coli* strain", respectively, and the three strains are collectively referred to as "OP27 site-specific mutant-expressing *E. coli* strains". They were stored as glycerol stocks until immediately before use.

Culture of the rhodopsin mutant-expressing *E*. *coli* strains was carried out as follows. Each of the OP27 site-specific mutant-expressing *E. coli* strains and the OP27-expressing *E. coli* strain from the glycerol stocks of them was subjected to static culture at 37°C in an LB (with addition of streptomycin) culture plate for 1 day, and three colonies formed were picked up and precultured in 5 mL of LB medium at 200 rpm and 37°C for 16 hours. The OP27 site-specific mutant-expressing *E*. *coli* strains and OP27-expressing *E. coli* strain, each in a volume of 0.4 mL, were each inoculated into 20 mL of LB medium, and cultured at 37°C and 200 rpm in a shaking incubator (BR-43FL, TAITEC CORPORATION, Japan). Once the OD₆₀₀ reached 0.4 to 1.0, 10 µM all-trans-retinal and 1 mM IPTG were added to each medium, and culture was performed at 37°C and 200 rpm for 3 hours.

The culture solutions collected were centrifuged at 5,000 × g and 25°C for 10 minutes to collect the OP27 site-specific mutant-expressing *E. coli* strains and OP27-expressing *E. coli* strain. The rhodopsin-expressing *E*. *coli* strains collected were each washed three times with 100 mM NaCl solution, and each suspended in 100 mM NaCl solution to reach OD₆₀₀ = 2. The suspensions of the rhodopsin-expressing *E. coli* strains were irradiated with light at 100 µE by using a 300-W halogen projector lamp (JCD100V-300W) equipped with a band-pass filter (PBO530-120, Asahi Spectra Co., Ltd., Japan: 530 ± 120 nm). The intensity of the light for irradiation was measured by using the photo-analyzer LA-105 (NK System, Osaka, Japan). The temporal variations of pH change in the suspensions of the OP27 site-specific mutant-expressing *E. coli* strains and OP27-expressing *E. coli* strain were recorded by using a pH meter (F-72, HORIBA, Ltd., Japan) connected to a PC. The proton translocation activities of the OP27-A31Q-expressing *E. coli* strain, OP27-I128G-expressing *E. coli* strain, OP27-A31Q/I128G-expressing *E. coli* strain, and OP27-expressing *E. coli* strain were determined from initial gradients of pH change. Proton transport per cell was determined by dividing proton translocation activity by cell concentration. The results for proton translocation activity and proton transport per cell were as shown in FIG. 2. It was suggested as shown in FIG. 2 that mutation resulted in increased proton translocation activity.

### Example 3: Artificial Design of OP27

OP27-A10, OP27-A20, OP27-A110, and OP27-A170 (SEQ ID NOs: 5 to 7, and 13, respectively), which were artificially designed mutants of the rhodopsin OP27, were each introduced into *E. coli.*

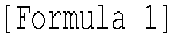

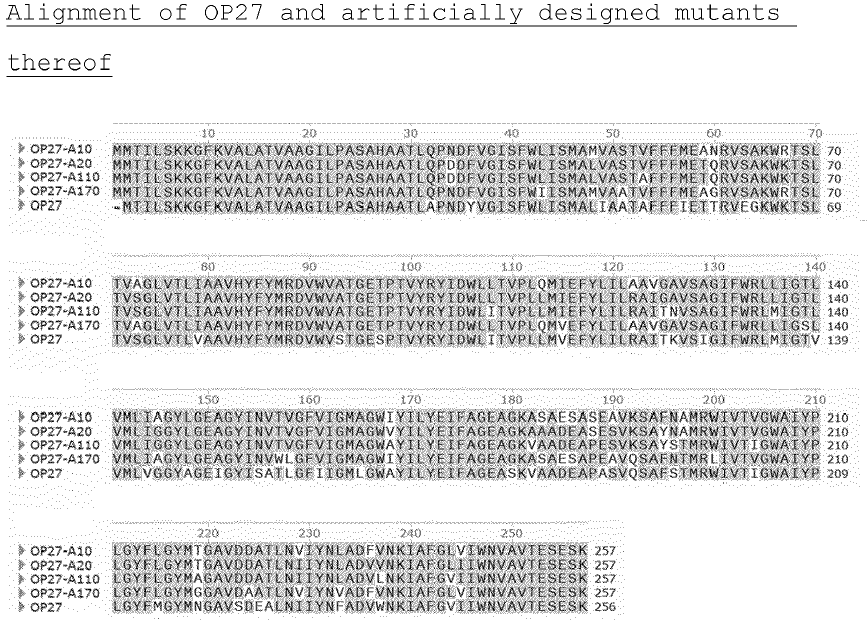

Specifically, the sequence of the restriction enzymes NdeI/KpnI was introduced into each of nucleic acids (DNAs) encoding OP27 and OP27-A10, OP27-A20, OP27-A110, and OP27-A170, and genes were artificially synthesized with codon optimization for *E. coli.* The synthesized OP27 gene and OP27-A10, OP27-A20, OP27-A110, and OP27-A170 genes were each introduced into a pCDF-Duet1 vector to prepare pCDF-OP27, pCDF-OP27-A10, pCDF-OP27-A20, pCDF-OP27-A110, and pCDF-OP27-A170. An *E. coli* MG1655(DE3) strain was transformed through the heat-shock method using pCDF-OP27 or pCDF-OP27-A10, pCDF-OP27-A20, pCDF-OP27-A110, or pCDF-OP27-A170. The *E. coli* strain obtained by introducing pCDF-OP27 into the *E. coli* MG1655 (DE3) strain is called "OP27-expressing *E. coli* strain", and was used as a control strain. The *E. coli* strains into which pCDF-OP27-A10, pCDF-OP27-A20, pCDF-OP27-A110, or pCDF-OP27-A170 was introduced are called "OP27-A10-expressing *E. coli* strain", "OP27-A20-expressing *E. coli* strain", "OP27-A110-expressing *E. coli* strain", and "OP27-A170-expressing *E. coli* strain", respectively, and the four strains are collectively referred to as "OP27 artificially designed mutant-expressing *E. coli* strains". They were stored as glycerol stocks until immediately before use.

Culture of the rhodopsin mutant-expressing *E*. *coli* strains was carried out as follows. Each of the OP27-expressing *E. coli* strain and the OP27 artificially designed mutant-expressing *E. coli* strains from the glycerol stocks of them was subjected to static culture at 37°C in an LB (with addition of streptomycin) culture plate for 1 day, and three colonies formed were picked up and precultured in 5 mL of LB medium at 200 rpm and 37°C for 16 hours. The OP27 artificially designed mutant-expressing *E. coli* strains and the OP27-expressing *E*. *coli* strain were each inoculated into 10 mL of M9 medium to reach an initial OD₆₀₀ of 0.2, and cultured at 37°C and 200 rpm in a shaking incubator (BR-43FL, TAITEC CORPORATION, Japan) in a dark place. Once the OD₆₀₀ reached 0.6, 10 µM all-trans-retinal and 20 µM isopropyl-β-thiogalactopyranoside were added to each medium, culture was performed in a dark place at 37°C and 200 rpm, and after the lapse of 3 hours, light irradiation was initiated with an LC-LED 450W (TAITEC CORPORATION, Japan) at ±50 µmol m⁻²s⁻¹ and culture was continued at 37°C and 200 rpm.

The OP27 artificially designed mutant-expressing *E. coli* strains and the OP27-expressing *E. coli* strain were collected after culturing for a proper period of time. Measurement was performed for cell concentration, proton translocation activity, and intracellular glutathione contents in the culture solutions. For each experiment condition, experiment was repeated three times.

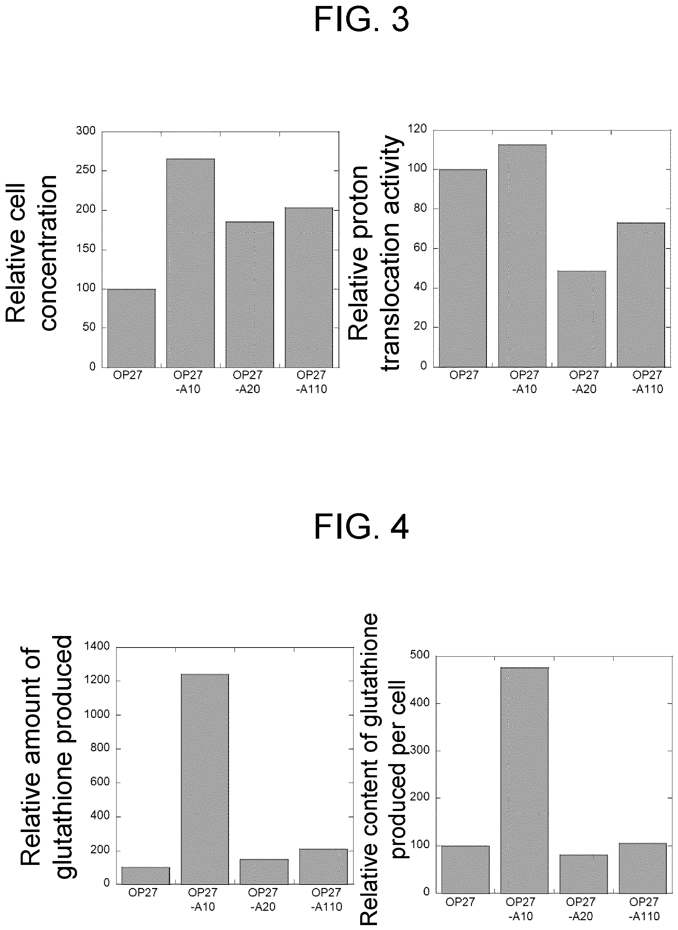

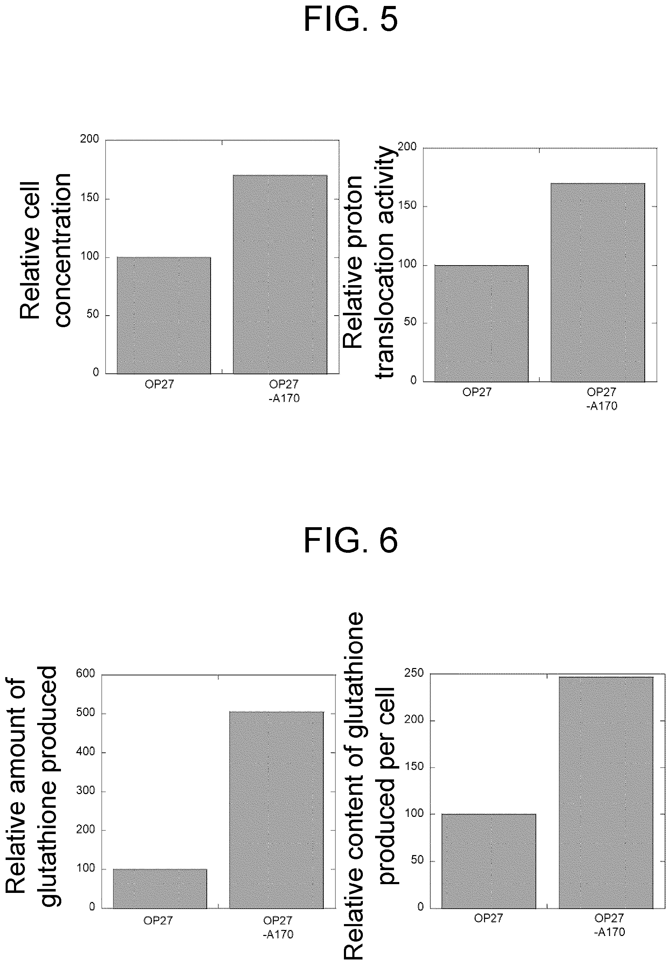

After main culture for 18 hours, each culture solution was collected and appropriately diluted, and the OD₆₀₀ was measured to determine the cell concentration. Separately, the culture solutions collected were centrifuged at 5,000 × g and 25°C for 10 minutes to collect the OP27 artificially designed mutant-expressing *E. coli* strains and the OP27-expressing *E. coli* strain. The rhodopsin-expressing *E. coli* strains collected were each washed three times with 100 mM NaCl solution, and each suspended in 100 mM NaCl solution to reach OD₆₀₀ = 2. The suspensions of the rhodopsin-expressing *E. coli* strains were irradiated with light by using a 300-W halogen projector lamp (JCD100V-300W) equipped with a band-pass filter (PBO530-120, Asahi Spectra Co., Ltd., Japan: 530 ± 120 nm). The intensity of the light for irradiation was measured by using the photo-analyzer LA-105 (NK System, Osaka, Japan). The temporal variations of pH change in the suspensions of the OP27 artificially designed mutant-expressing *E. coli* strains and the OP27-expressing *E. coli* strain were recorded by using a pH meter (F-72, HORIBA, Ltd., Japan) connected to a PC. The relative cell concentrations of a mutant-expressing *E*. *coli* strain in the cultures were determined. The proton translocation activities of the OP27-A10-expressing *E*. *coli* strain, OP27-A20-expressing *E. coli* strain, OP27-A110-expressing *E. coli* strain, and OP27-expressing *E*. *coli* strain were determined from initial gradients of pH change. FIG. 3 and FIG. 5 show the results. As shown in FIG. 3 and FIG. 5, OP27-A10 and OP27-A170 provided enhanced cell proliferative capacity and at the same time enhanced proton translocation activity. In contrast to these results, OP27-A20 and OP27-A110 provided enhanced cell proliferative capacity, but caused lowered proton translocation activity.

Each of the culture solutions collected through sampling was centrifuged at 7,000 × g for two minutes to separate cells. The cells were suspended in 50 mM Tris-HCl buffer (pH 8.0), and disrupted by using a cell disruptor (Shake Master NEO, Bio Medical Sciences) at 1500 rpm for 10 minutes, and centrifuged at 16,000 × g and 4°C for 10 minutes to remove disrupted cell fragments. The glutathione (GSH) concentration of the cell extract was measured by using 5,5'-dithiobis(2-nitrobenzoic acid)-glutathione reductase cycling assay described in Hara et al., 2009. Intracellular glutathione contents were determined by dividing glutathione concentration by cell concentration. The results were as shown in FIG. 4 and FIG. 6. As shown in FIG. 4 and FIG. 6, all the strains OP27-A10, OP27-A20, OP27-A110, and OP27-A170 provided enhanced glutathione production capacity, and OP27-A10 provided remarkably enhanced glutathione production capacity. OP27 is inferred to exhibit high proton translocation activity but have suppressive effect on cell proliferation. Although none of the OP27 mutants provided remarkably enhanced proton translocation activity, it is expected that the OP27 mutants cancelled suppression of cell proliferation to increase the total of (Number of cells) × (Proton transport per cell) and in turn increase the amount of ATP production, successfully leading to the increase in the energy available for material production.

The documents cited herein are totally incorporated herein by reference.

### Sequence Listing

SEQ ID NO: 1: Primer sequence
SEQ ID NO: 2: Primer sequence
SEQ ID NO: 3: Amino acid sequence of OP27
SEQ ID NO: 4: Amino acid sequence of OP27 mutant 1 (31, 128)
SEQ ID NO: 5: Amino acid sequence of OP27-A10 mutant
SEQ ID NO: 6: Amino acid sequence of OP27-A20 mutant
SEQ ID NO: 7: Amino acid sequence of OP27-A110 mutant
SEQ ID NO: 8: Amino acid sequence of OP27 mutant 2 (A10, A20, A110, A170)
SEQ ID NO: 9: Amino acid sequence of delta-rhodopsin (dR)
SEQ ID NO: 10: Amino acid sequence of E104 mutant
SEQ ID NO: 11: Amino acid sequence of E59 mutant
SEQ ID NO: 12: Amino acid sequence of delta-rhodopsin mutant (E104, E59)
SEQ ID NO: 13: Amino acid sequence of OP27-A170 mutant

## Claims

1. [A] A rhodopsin or a protein moiety thereof, wherein the rhodopsin has an amino acid sequence having 90% or higher identity with an amino acid sequence set forth in SEQ ID NO: 10 or 11 and is capable of exerting proton translocation activity higher than that of a rhodopsin having an amino acid sequence set forth in SEQ ID NO: 9;
[B] a rhodopsin or a protein moiety thereof, wherein the rhodopsin has an amino acid sequence having 90% or higher identity with an amino acid sequence set forth in any of SEQ ID NOs: 5 to 7 and 13 and is capable of exerting proton translocation activity higher than that of a rhodopsin having an amino acid sequence set forth in SEQ ID NO: 3; or
[C] a rhodopsin or a protein moiety thereof, wherein the rhodopsin has an amino acid sequence having 90% or higher identity with an amino acid sequence set forth in SEQ ID NO: 4, wherein the amino acid at position 31 of the amino acid sequence set forth in SEQ ID NO: 4 is Q and the amino acid at position 128 of the amino acid sequence set forth in SEQ ID NO: 4 is G, and is capable of exerting proton translocation activity higher than that of a rhodopsin having an amino acid sequence set forth in SEQ ID NO: 3.

2. A rhodopsin or a protein moiety thereof, wherein the rhodopsin:
(1) has an amino acid sequence set forth in SEQ ID NO: 12, and is capable of exerting proton translocation activity higher than that of a rhodopsin having an amino acid sequence set forth in SEQ ID NO: 9; or
(2) has an amino acid sequence set forth in SEQ ID NO: 4, and is capable of exerting proton translocation activity higher than that of a rhodopsin having an amino acid sequence set forth in SEQ ID NO: 3; or
(3) has an amino acid sequence set forth in SEQ ID NO: 8, and is capable of exerting proton translocation activity higher than that of a rhodopsin having an amino acid sequence set forth in SEQ ID NO: 3.

3. The rhodopsin according to claim 2 or a protein moiety thereof, wherein the rhodopsin has an amino acid sequence set forth in any of SEQ ID NOs: 4, 5 to 7, 10, 11, and 13.

4. A nucleic acid, encoding the protein moiety according to any one of claims 1 to 3.

5. A cell, comprising the rhodopsin according to any one of claims 1 to 3 or a protein moiety thereof.

6. A cell, comprising a nucleic acid encoding the protein moiety according to any one of claims 1 to 3.

7. A method for culturing the cell according to claim 5 or 6, the method comprising:
culturing the cell under culture conditions suitable for culture of the cell.

8. The method according to claim 7, wherein the culturing comprises culturing under light irradiation conditions.

9. A method for identifying or selecting an amino acid sequence or a nucleic acid sequence encoding the amino acid sequence, the method comprising:
(a1) selecting an opsin having proton translocation activity higher than that of an opsin having an amino acid sequence set forth in SEQ ID NO: 3 from amino acid sequences each having an amino acid sequence having 90% or higher identity with an amino acid sequence set forth in SEQ ID NO: 4, wherein the amino acid at position 31 of the amino acid sequence set forth in SEQ ID NO: 4 is Q and the amino acid at position 128 of the amino acid sequence set forth in SEQ ID NO: 4 is G, wherein the amino acid sequence having 90% or higher identity with the amino acid sequence set forth in SEQ ID NO: 4 is an amino acid sequence having one to several amino acid mutations, in particular, one or more mutations selected from the group consisting of insertion, deletion, substitution, addition, and removal from said amino acid sequence set forth in SEQ ID NO: 4;
(b1) selecting an opsin having proton translocation activity higher than that of an opsin having an amino acid sequence set forth in SEQ ID NO: 3 from amino acid sequences each having an amino acid sequence having 90% or higher identity with an amino acid sequence set forth in any of SEQ ID NOs: 5 to 7 and 13, wherein the amino acid sequence having 90% or higher identity with the amino acid sequence set forth in any of SEQ ID NOs: 5 to 7 and 13 is an amino acid sequence having one to several amino acid mutations, in particular, one or more mutations selected from the group consisting of insertion, deletion, substitution, addition, and removal from said amino acid sequence set forth in any of SEQ ID NOs: 5 to 7 and 13; or
(c1) selecting an opsin having proton translocation activity higher than that of an opsin having an amino acid sequence set forth in SEQ ID NO: 9 from amino acid sequences each having an amino acid sequence having 90% or higher identity with an amino acid sequence set forth in SEQ ID NO: 10 or 11, wherein the amino acid sequence having 90% or higher identity with the amino acid sequence set forth in SEQ ID NO: 10 or 11 is an amino acid sequence having one to several amino acid mutations, in particular, one or more mutations selected from the group consisting of insertion, deletion, substitution, addition, and removal from the amino acid sequence set forth in SEQ ID NO: 10 or 11.

10. A method for identifying or selecting an amino acid sequence or a nucleic acid sequence encoding the amino acid sequence, the method comprising:
(a2) selecting an opsin having proton translocation activity higher than that of an opsin having an amino acid sequence set forth in SEQ ID NO: 3 from an amino acid sequence set forth in SEQ ID NO: 4;
(b2) selecting an opsin having proton translocation activity higher than that of an opsin having an amino acid sequence set forth in SEQ ID NO: 3 from opsins each having an amino acid sequence having one or more mutations selected from the group consisting of (1) to (67) in an amino acid sequence set forth in SEQ ID NO: 8 by; or
(c2) selecting an opsin having proton translocation activity higher than that of an opsin having an amino acid sequence set forth in SEQ ID NO: 9 from an amino acid sequence set forth in SEQ ID NO: 12.
